# EUROPEAN PATENT APPLICATION

(11) **EP 1 462 106 A1**
(43) Date of publication of application: **29.09.2004**
(21) Application number: 03075904.7
(22) Date of filing: 28.03.2003
(51) Int. Cl.: A61K 31/565, A61K 31/57, A61P 15/00

(54) **Pharmaceutical compositions and kits comprising 17-beta-estradiol and a progesteron for the treatment of gynecological disorders**

(71) Applicant: Pantarhei Bioscience B.V., 3701 CH Zeist (NL)
(72) Inventor: Coelingh Bennink, Herman Jan Tijmen, 3971 BE Dribergen (NL); Visser, Monique, 3704 BB Zeist (NL)
(74) Representative: Jorritsma, Ruurd

(57) **Abstract**

The present invention is concerned with a method of treating or preventing an estrogen sensitive gynaecological disorder in a female mammal, said method comprising administering to the female a combination of estrogen and progestogen continuously for at least 3 months, wherein the estrogen is selected from the group consisting of 17β-estradiol, precursors of 17β-estradiol and combinations thereof, said estrogen being administered in an amount equivalent to a daily oral dosage of 2.2-5 mg 17β-estradiol and said progestogen being administered in an amount equivalent to a daily oral dose of 5-50 mg dydrogesterone.

Another aspect of the invention relates to a kit comprising a plurality of oral dosage units, said plurality of daily hormone units containing an estrogen in an amount equivalent to 2.2-5 mg 17β-estradiol and a progestogen in an amount equivalent to 5-50 mg dydrogesterone, wherein the estrogen is selected from the group consisting of 17β-estradiol, precursors of 17β-estradiol and combinations thereof.

## Description

### TECHNICAL FIELD OF THE INVENTION

The present invention is concerned with a new method of treating or preventing an estrogen sensitive gynaecological disorder in a female mammal. More particularly the present invention relates to such a method comprising administering to the female a combination of estrogen and progestogen continuously for at least 3 months. The present method is particularly suitable for treating an estrogen sensitive gynaecological disorder selected from the group consisting of endometriosis, endometrial hyperplasia, endometrial cancer, uterine leiomyomas, adenomyosis, dysmenorrhoea, menorrhagia and metrorrhagia.

The invention also relates to a pharmaceutical kit comprising a plurality of oral dosage units, said plurality of daily hormone units containing an estrogen and a progestogen.

### BACKGROUND OF THE INVENTION

Endometriosis is one of the most common gynaecological disorders, affecting 10 to 15% of women in the reproductive age. It is a benign disease defined as the presence of viable endometrial gland and stroma cells outside the uterine cavity, and is most frequently found in the pelvic area. In women developing endometriosis, these endometrial cells have the capacity to adhere to and invade the peritoneal lining, and are then able to implant and grow. It is not known yet why some women develop endometriosis and others do not. The implants respond to the menstrual cycle in a similar way as the endometrium in the uterus. However, infiltrating lesions and the blood from these lesions, unable to leave the body, cause inflammation of the surrounding tissue. The most common symptoms of endometriosis are dysmenorrhoea, dyspareunia and (chronic) abdominal pain. The occurrence of these symptoms is not related to the extent of the lesions. Some women with severe endometriosis are asymptomatic, while women with mild endometriosis may have severe pain.

Until now, no non-invasive test is available to diagnose endometriosis. Laparoscopy has to be performed to diagnose the disease. Endometriosis is classified according to the 4 stages set up by the American Fertility Society (AFS). Stage I corresponds to minimal disease while stage IV is severe, depending on the location and the extent of the endometriosis.

Endometriosis is found in up to 50% of the women with infertility. However, currently no causal relation is known to exist between mild endometriosis and infertility. Moderate to severe endometriosis can cause tubal damage and adhesions leading to infertility.

Despite extensive research, the cause of endometriosis is still largely unknown. Several theories for the origin of endometriosis have been proposed, although no single hypothesis explains all cases of the disease completely. However, the key event in all these theories is the occurrence of retrograde menstruation.

The aims of treatment of endometriosis are pain relief, resolution of the endometriotic tissue and restoration of fertility (if desired). The two common treatments are surgery or hormonal therapy or a combination of both. Surgical treatment removes the endometriotic tissue. Initially, the pain relief using this procedure approaches 70-80%. However, the pain returns in a lot of cases because of re-growth of the endometriotic tissue. At present the most permanent way to treat endometriosis is the removal of the ovaries, thus eliminating the production of estrogens and possible other ovarian factors, which regulate the growth and activity of the endometriotic tissue.

The currently available pharmacological treatments of endometriosis are antiinflammatory and hormonal. In the early stages of endometriosis non-steroidal antiinflammatory drugs (NSAID's) are often successful in relieving the pelvic pain. Hormonal treatment is given mainly to down-regulate the estrogen production by the ovaries. Various drugs are available for suppressing this ovarian function as will be explained below.

Danazol and gestrinone are both testosterone-derivatives, suppressing the pituitary release of follicle stimulating hormone (FSH) and luteinising hormone (LH). The efficacy of these two drugs on the regression of endometriotic tissue is no better than that of other hormonal treatments. However, both these drugs have pronounced androgenic side effects, like weight gain, acne and hirsutism, which explains the diminishing popularity of these drugs. In addition, these drugs produce a hypoestrogenic milieu.

Gonadotrophin releasing hormone (GnRH) agonists (e.g. nafareline, busereline) give a more complete suppression of the ovarian activity, leading to down-regulation of LH and FSH receptors. However, this inactivity of the ovaries does not result in disappearance of the endometriosis. Several comparative randomised studies have shown that the efficacy of these drugs is no better than that of other existing hormonal treatments. The use of GnRH agonists is limited because the women taking these drugs develop hypoestrogenic symptoms, such as hot flushes, sweating, headache, vaginal dryness, and decrease in bone mineral density (BMD). Therefore these drugs can only be administered for a maximal period of approximately 6 months. Add-back therapy (suppletion of low doses of an estrogen, an estrogen with a progestogen or a progestogen with estrogenic activity) is sometimes given to women receiving GnRH agonists, to diminish the hypoestrogenic symptoms. However, it still has to be proven if treatment with GnRH agonists and add-back therapy for more than 6 months without unwanted side effects is possible and if such a treatment remains effective against endometriosis throughout said period.

Progestogens have been used in a wide range of pharmaceutical applications for decades, including endometriosis. These drugs also work by suppressing LH and FSH and consequently induce hypoestrogenism. Examples of progestogens given for endometriosis are medroxyprogesterone acetate, dydrogesterone and lynestrenol. These drugs are also associated with side-effects e.g. mood changes and breakthrough bleedings. The treatment of endometriosis with progestogens has not received regulatory approval in the United States.

A large percentage of women experience relief of symptoms while being treated with the above hormonal drugs. However, symptom recurrence is likely once the drug is discontinued. None of the aforementioned drugs is suitable for long term treatment of endometriosis, because of the severe side-effects which are largely associated with hypoestrogenism. These treatments are therefore in most cases discontinued after a period of 6 months after which recurrence of the symptoms is likely to occur.

It will be evident from the above that there is a great need for a pharmaceutical treatment of endometriosis, which treatment may be applied for a longer period of time than the existing hormonal treatments, preferably until such time that the treated female reaches menopause, and/or which treatment produces better results, particularly in terms of side-effects during treatment and recurrence rate after discontinuation of the therapy.

Oral contraceptives, containing both an estrogen (ethinyl estradiol) and progestogen, are also prescribed for endometriosis. However, this treatment is not optimal, because contraceptive methods are accompanied with recurring withdrawal bleedings that also causes bleeding in endometriotic tissue. It is known that such withdrawal bleedings may be avoided by continuous combined administration of estrogen and progestogen. However, these so called continuous combined regimens are known to give rise to unscheduled bleeding and spotting. Unscheduled bleeding and spotting in females suffering from estrogen sensitive gynaecological disorders not only is a nuisance but also can be quite painful. Furthermore, there are reasons to believe that spotting and bleeding will accelerate the progression of such disorders.

Everything that has been said above in relation to the treatment of endometriosis equally applies to other benign estrogen sensitive gynaecological disorders, notably adenomyosis, uterine fibroids, dysmenorrhoea, menorrhagia and metrorrhagia as well as to endometrial hyperplasia and endometrial cancer. These gynaecological disorders are all estrogen sensitive and treated in a comparable way as described herein before in relation to endometriosis. The available pharmaceutical treatments, however, suffer from the same major drawbacks as mentioned in connection with endometriosis.

### SUMMARY OF THE INVENTION

The inventors have surprisingly found that continuous combined administration of a relatively high dose of 17β-estradiol (E2) in combination with a progestogen very effectively suppresses endometrial thickening. The condition of amenorrhoea induced by the present method in combination with the absence of significant endometrial growth has a pronounced beneficial impact on gynaecological disorders such as endometriosis, endometrial hyperplasia, endometrial cancer, uterine leiomyomas, adenomyosis, dysmenorrhoea, menorrhagia and metrorrhagia. It is very unexpected that, despite the use of a high dose of E2, the present method does not give rise to significant endometrial stimulation, particularly because treatment of e.g. endometriosis with a combination of a relatively low dose of E2 and a progestogen is associated with significant endometrial thickening.

An important advantage of the present method resides in the fact that it causes significantly less unscheduled bleeding and spotting than continuous combined treatment with known oral contraceptives or with a combination of a low dose of E2 and a progestogen. Also, the present invention employs the endogenous natural estrogen E2, whereas commercially available oral contraceptives are based on synthetic estrogens such as ethinyl estradiol.

In comparison to other known methods of treating estrogen sensitive gynaecological disorders that are based on inhibiting endogenous production of E2, the present method offers the important advantage that it will not induce hypoestrogenism. Therefore, unlike these methods from the prior art, the present method may suitably be continued for an indefinite period of time.

### DETAILED DESCRIPTION OF THE INVENTION

Accordingly, one aspect of the invention is concerned with a method of treating or preventing a gynaecological disorder in a female mammal, said method comprising administering to the female a combination of estrogen and progestogen continuously for at least 3 months, wherein the estrogen is selected from the group consisting of 17β-estradiol, precursors of 17β-estradiol and combinations thereof, said estrogen being administered in an amount equivalent to a daily oral dosage of 2.2-5 mg 17β-estradiol and said progestogen being administered in an amount equivalent to a daily oral dose of 5-50 mg dydrogesterone. Examples of gynaecological disorders that may suitably be treated or prevented by the present method include endometriosis, endometrial hyperplasia, endometrial cancer, uterine leiomyomas, adenomyosis, dysmenorrhoea, menorrhagia and metrorrhagia. Preferably the present method is used in the treatment or prevention of endometriosis or endometrial hyperplasia. Most preferably the method is used to treat or prevent endometriosis.

The present method may suitable employ any pharmaceutically acceptable substance with sufficient progestogenic activity. The present invention encompasses the use of substances that exhibit progestogenic activity *per se* as well as precursors of such substances. The invention also encompasses the use of progestogen metabolites that exhibit progestogenic activity. Examples of progestogens that may suitably be employed in accordance with the invention include dydrogesterone, norethisterone, levonorgestrel, norgestimate, drospirenone, 3-beta-hydroxydesogestrel, 3-keto desogestrel, 17-deacetyl norgestimate, 19-norprogesterone, acetoxypregnenolone, allylestrenol, anagestone, chlormadinone, cyproterone, demegestone, desogestrel, dienogest, dihydrodydrogesterone, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol, medrogestone, medroxyprogesterone, megestrol, melengestrol, nomegestrol, norethindrone (=norethisterone), norethynodrel, norgestrel, norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, precursors of these progestogens and combinations thereof. Preferably, the progestogen is selected from the group consisting of dydrogesterone, dihydrodydrogesterone, norethisterone, levonorgestrel, desogestrel, norgestimate, drospirenone, cyproterone, gestodene, trimegestone, progesterone, precursors of these progestogens and combinations thereof. Even more preferably, the progestogen is selected from the group consisting of dydrogesteron, dihydrodydrogesterone, norethisterone, precursors of these progestogens and combinations thereof. Most preferably, the progestogen is dydrogesterone, dihydrodydrogesterone or a precursor thereof.

The present method may suitably employ a precursor of 17β-estradiol or a precursor of the progestogen. Such precursors are capable of liberating 17β-estradiol or a progestogen when used in the present method, e.g. as a result of metabolic conversion. Examples of suitable precursors of 17β-estradiol and progestogens include such substances wherein the hydrogen atom of at least one of the hydroxyl groups has been substituted by an acyl radical of a hydrocarbon carboxylic, sulfonic acid or sulfamic acid of 1-25 carbon atoms; tetrahydrofuranyl; tetrahydropyranal; or a straight or branched chain glycosidic residue containing 1-20 glycosidic units per residue.

Typical examples of precursors which can suitably be used in accordance with the invention are esters that can be obtained by reacting the hydroxyl groups of the estrogenic substances with substances that contain one or more carboxy (M^{+ -}OOC-) groups, wherein M⁺ represents a hydrogen or (akali)metal cation. Hence, in a particularly preferred embodiment, the precursors are derivatives of 17β-estradiol or a progestogen, wherein the hydrogen atom of at least one of the hydroxyl groups has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

A critically important advantage of the present method resides in the fact that it suppresses endometrial growth. Typically, in the present method, the continuous administration of the combination of estrogen and progestogen is effective in maintaining a substantially constant thin endometrium with a thickness of less than 8 mm, preferably of less than 6 mm.

The continuous administration of the combination of estrogen and progestogen is advantageously continued for a period of at least 4 months, more preferably for a period of at least 6 months. Most preferably, the present method employs continuous combined administration of the two steroids for at least 9 months.

The term "continuous" as used in here, means that the combination of estrogen and progestogen is administered at relatively regular intervals, with no (therapeutically) significant interruptions. Naturally, minor interruptions may occur that do not affect the overall effectiveness of the present method, and indeed such aberrations are encompassed by the present invention. In a preferred embodiment, and more arithmetically, the administration regimen is deemed to be continuous if the longest interval between 2 subsequent administrations is not more than 3.5 times as long as the average interval. Even more preferably said longest interval is not more than 2.5 times, most preferably not more than 1.5 times as long as the average interval.

The present method requires continuous administration of the combination of estrogen and progestogen for a period of at least 3 months. In order to minimise the risk of so called breakthrough bleeding, it can be advantageous to periodically interrupt the continuous administration for a period of 4-12 days, preferably for a period of 5-9 days. These interruptions will cause a predictable withdrawal bleeding, following which continuous administration of the combination of estrogen and progestogen can be resumed with a reduced risk of breakthrough bleeding. Preferably, such an interruption occurs after 3-12 months of continuous administration, more preferably after 3-8 months and most preferably after 3-6 months of continuous administration. In a particularly preferred embodiment, the present method employs a protocol with at least 2 interruptions following periods of continuous combined administration.

As explained before, it is a critical element of the present invention to administer a relatively high dose of estrogen. In a particularly preferred embodiment the estrogen is administered in an amount equivalent to a daily oral dosage of at least 2.4 mg 17β-estradiol, more preferably of at least 2.5 mg and most preferably of at least 2.6 mg 17β-estradiol. Preferably, the estrogen is administered in a dosage that does not exceed an amount equivalent to a daily oral dosage of 4.5 mg 17β-estradiol, more preferably it does not exceed an amount equivalent to a daily oral dosage of 4 mg 17β-estradiol and most preferably, it does not exceed an amount equivalent to a daily oral dosage of 3.5 mg 17β-estradiol.

In another preferred embodiment of the present method the progestogen is administered in an amount equivalent to a daily oral dosage of 8-30 mg dydrogesterone, more preferably in an amount equivalent to a daily oral dosage of 10-20 mg dydrogesterone. The equivalent daily oral dosages for the progestogen norethisterone acetate may be calculated by dividing the recommended dosages for dydrogesterone by 10. Similarly, equivalent dosages for other progestogens may be obtained by using conversion factors that are known in the art or that may be established by methods well known to the person skilled in the art.

The amount of estrogen administered in accordance with the present method is preferably effective to achieve a 17β-estradiol serum concentration of at least 30 pg/ml, more preferably of at least 50 pg/ml.

In a preferred embodiment of the invention the estrogen and progestogen are administered orally, transdermally, subcutaneously, intravaginally or intrauterinely. Although it is feasible to use different modes of administration for the estrogen and the progestogen, it is preferred to administer both components in the same way and essentially simultaneously. For convenience sake it is advisable to administer both components by means of a single formulation. Most preferably the present method employs oral administration of the estrogen and progestogen.

The combination of estrogen and progestogen is advantageously administered at least once daily so as to maintain a relatively constant serum concentration. Most preferably the combination is administered once daily.

The present method is particularly suitable for treating humans, primates, bovines, porcines, equines, canines or felines. Most advantageously the present method is employed in the treatment of humans.

It was found that the present method is particularly advantageous in non-smoking females. Although the inventors do not wish to be bound by theory it is believed that smoking interferes with E2-metabolisation in a way that reduces the bioavailability of E2, e.g. by increasing 2-hydroxylation of E2. Thus, the present method is preferably employed to prevent conception in a non-smoking human female.

It was surprisingly found that the anti-proliferative effect of the present combination of estrogen and progestogen on the endometrium may be enhanced further by co-administration of an androgen. In addition, the continuous co-administration of an androgen in the present regimen offers the advantage of even less vaginal breakthrough spotting and bleeding. Consequently, in another preferred embodiment of the invention, the present method comprises co-administration of an androgen.

Preferably, in the present method, androgen is co-administered in an amount effective to maintain the serum androgen concentration of the female mammal at a level equivalent to between 0.5 and 5.0, preferably between 0.7 and 4.0 and most preferably between 1.0 and 3.0 nanomoles testosterone per litre. Here the testosterone concentrations relate to the total testosterone present in the serum, i.e. including both free testosterone and bound testosterone.

The androgen used in the present method is preferably selected from the group consisting of dehydroepiandrosterone (DHEA); DHEA-sulphate; testosterone; testosterone esters such as testosterone undecanoate , testosterone propionate, testosterone phenylpropionate, testosterone isohexanoate, testosterone enantate, testosterone bucanate, testosterone decanoate, testosterone buciclate; danazol; gestrinone; methyltestosterone; mesterolon; stanozolol; androstenedione; dihydrotestosterone; androstanediol; metenolon; fluoxymesterone; oxymesterone; methandrostenolol; 7α-methyl-19-nortestosterone (MENT); precursors capable of liberating these androgens when used in the present method and mixtures thereof. Most preferably the androgen is selected from the group consisting of DHEA, danazol, gestrinone, testosterone esters, androstenedione, precursors capable of liberating these androgens when used in the present method and mixtures thereof. Preferably the testosterone esters employed in the present method comprise an acyl group which comprises at least 6, more preferably from 8-20 and preferably 9-13 carbon atoms. Most preferably the androgens used in the present method are DHEA and/or testosterone undecanoate. These androgens offer the advantage that they can effectively be used in oral dosage units.

It is noted that, for instance, DHEA, testosterone undecanoate and androstenedione are precursors of testosterone and that said precursors *per se* exhibit virtually no affinity for androgen receptors in the female body. The effectiveness of the androgens within the method of the invention is determined by their functionally active form, which may well be different from the form in which they are administered.

In a preferred embodiment the androgen is provided in an amount equivalent to a daily oral dosage of 5 to 250 mg DHEA, which is equivalent to a daily oral dosage of 1 to 50 mg testosterone undecanoate. More preferably the androgen is provided in an amount which is equivalent to a daily oral dosage of 10-120 mg DHEA. Most preferably the androgen is administered in an amount which is equivalent to a daily oral dosage of 20-60 mg DHEA.

Another aspect of the invention relates to a kit comprising a plurality of oral dosage units, said plurality of daily hormone units containing an estrogen in an amount equivalent to 2.2-5 mg 17β-estradiol and a progestogen in an amount equivalent to 5-50 mg dydrogesterone, wherein the estrogen is selected from the group consisting of 17β-estradiol, precursors of 17β-estradiol and combinations thereof. Because the present kit is intended for use in a method that employs continuous combined administration, said kit preferably does not contain dosage units that do not contain a combination of estrogen and progestogen. Even more preferably, the kit does not contain any dosage units that do not contain the combination of estrogen and the progestogen in the indicated amounts.

Typically, the present kit contains at least 40 dosage units, preferably at least 60 and more preferably at least 100 dosage units. These dosage units may be held in a container, or alternatively they may be held in a strip from which they may be removed individually by the user.

The invention is further illustrated by means of the following examples.

### EXAMPLES

### Example 1

A clinical study was conducted in 30 young healthy female volunteers (15 smokers and 15 non-smokers) who were using a combined monophasic oral contraceptive with at least 30 microgram ethinyl estradiol at the moment of enrolment.

Immediately following 19-25 days of taking this monophasic oral contraceptive, without observing a tablet-free period, treatment with the study medication was commenced. The study medication comprised of continuously administering 3 mg 17beta-estradiol (E2) combined with 1.5 mg norethisterone acetate without pauses for 3 months. Follicular development and endometrial thickness were measured by ultrasonography once every 3 or 4 days depending on follicle growth. Vaginal spotting and bleeding was scored daily by the participants in a diary. In addition, endocrine measurements (E2 and progesterone) were performed in a subgroup of the participants (4 individuals who exhibited follicular growth, 5 randomly selected non-smokers and 5 randomly selected smokers). Specific contraceptive side-effects, mood changes and volunteer appreciation were also evaluated.

No ovulations were observed in any of the participants. In 4 of the 30 participants (2 smokers and 2 non-smokers) persistent follicles of 25-30 mm were seen. In these women, no increase in the progesterone levels was observed. No dominant follicles were seen in the other 26 women (median diameter 5 mm). Endometrial thickness did not change during treatment (median 4.5 mm before and 4.0 mm after treatment). After an initial adaptation period spotting was found to occur infrequently in 3 out of 15 non-smokers. In smokers however bleeding and spotting occurred in 3 out of 15 women and spotting only in another 5 women. In some women breast tension, bloating and acne occurred during treatment. No mood changes were registered. Of the 30 participating women, 12 would use this contraceptive when available and 12 would consider its use.

### Example 2

Example 1 is repeated with the exception that the participants continuously receive 3 mg 17beta-estradiol (E2) combined with 15 mg dydrogesterone without pauses for 4 months. Similar results are obtained as described in Example 1.

### Example 3

The study described in Example 2 is repeated with the exception that after the period of 4 months of continuous combined administration, the participants receive placebo's during 7 subsequent days, resulting in withdrawal bleeding in all subjects. Immediately following these 7 days, the participants again receive the combination of E2 and dydrogesterone uninterruptedly for another 4 months. Following this period of 4 months the women yet again receive placebo's during 7 subsequent days. Also this time, withdrawal bleeding is observed in all participants during the placebo period.

In parallel, another group of 30 women, which women were selected on the basis of the same criteria mentioned in Example 1, receives the combination of E2 and dydrogesterone continuously, i.e. without interruptions, during period of 8 months and 14 days.

It was found that the incidence of bleeding and spotting in the latter group was significantly higher than in the group that alternately received the combination of E2 and dydrogesterone for 4 months followed by an administration-free interval of 7 days.

## Claims

1. Use of an estrogen in the manufacture of a pharmaceutical preparation for use in a method of treating or preventing an estrogen sensitive gynaecological disorder in a female mammal, said method comprising administering to the female a combination of estrogen and progestogen continuously for at least 3 months, wherein the estrogen is selected from the group consisting of 17β-estradiol, precursors of 17β-estradiol and combinations thereof, said estrogen being administered in an amount equivalent to a daily oral dosage of 2.2-5 mg 17β-estradiol and said progestogen being administered in an amount equivalent to a daily oral dose of 5-50 mg dydrogesterone.

2. Use according to claim 2, wherein the gynaecological disorder is selected from the group consisting of endometriosis, endometrial hyperplasia, endometrial cancer, uterine leiomyomas, adenomyosis, dysmenorrhoea, menorrhagia and metrorrhagia.

3. Use according to claim 1 or 2, wherein the progestogen is selected from the group consisting of dydrogesterone, norethisterone, levonorgestrel, norgestimate, drospirenone, 3-beta-hydroxydesogestrel, 3-keto desogestrel, 17-deacetyl norgestimate, 19-norprogesterone, acetoxypregnenolone, allylestrenol, anagestone, chlormadinone, cyproterone, demegestone, desogestrel, dienogest, dihydrodydrogesterone, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol, medrogestone, medroxyprogesterone, megestrol, melengestrol, nomegestrol, norethindrone (=norethisterone), norethynodrel, norgestrel, norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, precursors of these progestogens and combinations thereof.

4. Use according to claim 3, wherein the progestogen is selected from the group consisting of dydrogesterone, dihydrodydrogesterone, norethisterone, levonorgestrel, desogestrel, norgestimate, drospirenone, cyproterone, gestodene, trimegestone, progesterone, precursors of these progestogens and combinations thereof.

5. Use according to claim 4, wherein the progestogen is dydrogesterone, dihydrodydrogesterone or a precursor thereof.

6. Use according to any one of the preceding claims, wherein the precursor of 17β-estradiol is an ester of 17β-estradiol.

7. Use according to any one of claims 3-5, wherein the precursor of the progestogen is an ester of said progestogen.

8. Use according to any one of the preceding claims, wherein the continuous administration of the combination of estrogen and progestogen is effective in maintaining a substantially constant endometrial thickness of less than 8 mm, preferably of less than 6 mm.

9. Use according to any one of the preceding claims, wherein the estrogen is administered in an amount equivalent to a daily oral dosage of 2.4-4 mg 17β-estradiol, preferably to a daily oral dosage of 2.5-3.5 mg 17β-estradiol.

10. Use according to any one of the preceding claims, wherein the progestogen is administered in an amount equivalent to a daily oral dosage of 8-30 mg dydrogesterone, preferably to a daily oral dosage of 10-20 mg dydrogesterone.

11. Use according to any one of the preceding claims, wherein the estrogen is administered in an amount effective to achieve a 17β-estradiol serum concentration of at least 30 pg/ml.

12. Use according to any one of the preceding claims, wherein the estrogen and progestogen are administered orally, transdermally, subcutaneously, intravaginally or intrauterinely.

13. A kit comprising a plurality of oral dosage units, said plurality of daily hormone units containing an estrogen in an amount equivalent to 2.2-5 mg 17β-estradiol and a progestogen in an amount equivalent to 5-50 mg dydrogesterone, wherein the estrogen is selected from the group consisting of 17β-estradiol, precursors of 17β-estradiol and combinations thereof.

14. A kit according to claim 13, wherein the kit does not contain dosage units that do not contain the estrogen and the progestogen in the indicated amounts.

15. A kit according to claim 13 or 14, wherein the kit contains at least 40 dosage units, preferably at least 100 dosage units.
